(19) 

**Europäisches Patentamt**

**European Patent Office**

**Office européen des brevets**

(11) **EP 4 380 520 B1**

(12) **EUROPEAN PATENT SPECIFICATION**

(45) Date of publication and mention
of the grant of the patent:
**06.05.2026  Bulletin 2026/19**

(21) Application number: **22748443.3**

(22) Date of filing: **13.07.2022**

(51) International Patent Classification (IPC):
***A61F 9/007*** *(2006.01)*

(52) Cooperative Patent Classification (CPC):
**A61F 9/00745**

(86) International application number:
**PCT/IB2022/056468**

(87) International publication number:
**WO 2023/017334 (16.02.2023 Gazette 2023/07)**

(54) **ELECTRONICALLY DETECTING PHACOEMULSIFICATION TIP ENGAGEMENT WITH A LENS**

ELEKTRONISCHE DETEKTION DER EINRASTUNG EINER PHAKOEMULSIFIKATIONSSPITZE
MIT EINER LINSE

DÉTECTION ÉLECTRONIQUE D'ENGAGEMENT DE POINTE DE PHACOÉMULSIFICATION AVEC
UNE LENTILLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.08.2021  US 202163230741 P
26.04.2022  US 202217729343**

(43) Date of publication of application:
**12.06.2024  Bulletin 2024/24**

(73) Proprietor: **Johnson & Johnson Surgical Vision,
Inc.
Irvine, CA 92618 (US)**

(72) Inventors:
 • **GOVARI, Assaf
  2066717 Yokneam (IL)**
 • **BEECKLER, Christopher Thomas
  Irvine, California 92618 (US)**

 • **GLINER, Vadim
  2066717 Yokneam (IL)**
 • **SITNITSKY, Ilya
  2066717 Yokneam (IL)**
 • **FUCHS, Amit
  2066717 Yokneam (IL)**
 • **KEYES, Joseph Thomas
  Irvine, California 92618 (US)**
 • **AHARON, Eran
  2066717 Yokneam (IL)**

(74) Representative: **Carpmaels & Ransford LLP
One Southampton Row
London WC1B 5HA (GB)**

(56) References cited:
**WO-A1-92/11814     US-A1- 2013 211 435
US-A1- 2015 045 806     US-A1- 2015 216 726
US-A1- 2016 175 543     US-A1- 2021 196 515**

**Description**

**FIELD OF THE DISCLOSURE**

The present disclosure relates generally to systems and probes that utilize piezoelectric vibration, and particularly to phacoemulsification systems and probes.

**BACKGROUND OF THE DISCLOSURE**

A cataract is a clouding and hardening of the eye's natural lens, a structure which is positioned behind the cornea, iris, and pupil. The lens is mostly made up of water and protein and as people age these proteins change and may begin to clump together obscuring portions of the lens. To correct this, a physician may recommend phacoemulsification cataract surgery. In the procedure, the surgeon makes a small incision in the sclera or cornea of the eye. Then a portion of the anterior surface of the lens capsule is removed to gain access to the cataract. The surgeon then uses a phacoemulsification probe, which has an ultrasonic handpiece with a needle. The tip of the needle vibrates at ultrasonic frequency to sculpt and emulsify the cataract while a pump aspirates particles and fluid from the eye through the tip. Aspirated fluids are replaced with irrigation of a balanced salt solution to maintain the anterior chamber of the eye. After removing the cataract with phacoemulsification, the softer outer lens cortex is removed with suction. An intraocular lens (IOL) is then introduced into the empty lens capsule restoring the patient's vision.

Various techniques to control an ultrasonic transducer of a surgical tool were proposed in the patent literature. For example, International Patent Application Publication WO 2021/119616 describes systems and methods for controlling vibrations of an ultrasonic handpiece that generates an AC drive signal applied to a transducer of the ultrasonic handpiece to vibrate a tip of the ultrasonic handpiece. A property relating to a stiffness of tissue being contacted by the vibrating tip is determined based on a measured voltage and a measured current of the AC drive signal. A target displacement for the tip is determined based on the tissue property, and the AC drive signal is adjusted to achieve the determined target displacement. WO9211814A1 describes an apparatus for operating on the human eye which detects changes in load, more specifically, mechanical impedance, of a transducer and controls aspiration based on the load changes.

The present disclosure will be more fully understood from the following detailed description of the examples thereof, taken together with the drawings in which:

**BRIEF DESCRIPTION OF THE DRAWINGS**

Fig. 1 is a pictorial view of a phacoemulsification system constructed to operate in accordance with an example of the present disclosure;
Fig. 2 is a block diagram schematically illustrating a multi-channel piezoelectric drive system, in accordance with an example of the present disclosure;
Fig. 3 is a schematic graph showing the electrical impedance magnitude of an ultrasonic transducer as a function of driving frequency for a given mechanical load, in accordance with an example of the present disclosure; and
Fig. 4 is a flow chart describing steps providing a physician with an indication that a needle of a phacoemulsification probe has contacted an eye lens of a patient, in accordance with examples of the present disclosure.

**SUMMARY**

The invention provides a system as defined by the appended independent claims, with certain embodiments defined in the dependent claims. Where methods of treatment by surgery are described below, these are provided for context and understanding of the invention, but the methods themselves do not form part of the claimed invention.

**DETAILED DESCRIPTION OF EXAMPLES**

OVERVIEW

A phacoemulsification system typically drives a piezoelectric actuator included in a phacoemulsification probe/handpiece to vibrate a needle of a phacoemulsification probe during a cataract procedure. The piezoelectric actuator of the phacoemulsification probe may be designed to vibrate, in resonance, in a single mode or in multiple modes simultaneously, where each mode has a given "natural" resonant frequency. At resonance, the actuator electrical impedance experienced by a driving circuitry is minimal. However, the resonance frequency may drift during phacoemulsification, for example due to change in mechanical loads on the vibrating needle, which thereby changes the experienced electrical impedance. An important function of an ultrasonic driver circuitry is to locate and track the desired resonance frequency.

Examples of the present disclosure that are described herein provide a technique for detecting vibrating needle engagement with the lens, and disengagement of the needle from the lens, by measuring electrical impedances seen by one or more electronic modules driving one or more respective piezoelectric crystals vibrating the needle. The needle is typically configured to be in mechanical resonance, but as noted above, the resonant frequency changes according to the mass of matter in the eye that couples with the needle. A change in mechanical resonant frequency changes (e.g., increases) the electrical impedances seen by the one or more driving modules. A processor uses this change in impedance to provide an indication of the change to a handpiece user. The indication may indicate engagement with or disengagement from the lens. The method may also be used to detect other changes, such as changes in viscosities of elements close to the needle.

Examples of indication include audio and/or visual notifications of the needle assuming contact with the eye lens or disengaging the lens, such as a sound, a blipping color, and a worded sound notification or worded visual notification. Another example of an indication is by vibration of, for example, an element in the handpiece handle.

In another example, in addition to notifying of the change to the handpiece user, the processor may change a driving signal level in response to the change, such as reducing vibration power to lower a level of vibration of the needle when disengagement from the lens is detected. Alternatively, no indication may be provided to the user, but rather the processor may make a change (e.g., automatically) to a drive signal level or frequency adjustment to an electrical resonant frequency in response to the change detected (e.g., detecting engagement/disengagement of lens material from the needle). In an example, upon detection of a change indicating engagement of lens material with the needle, the vibration of the needle may be activated and upon detection of a change indicate disengagement of lens material from the needle, the vibration of the needle may be deactivated.

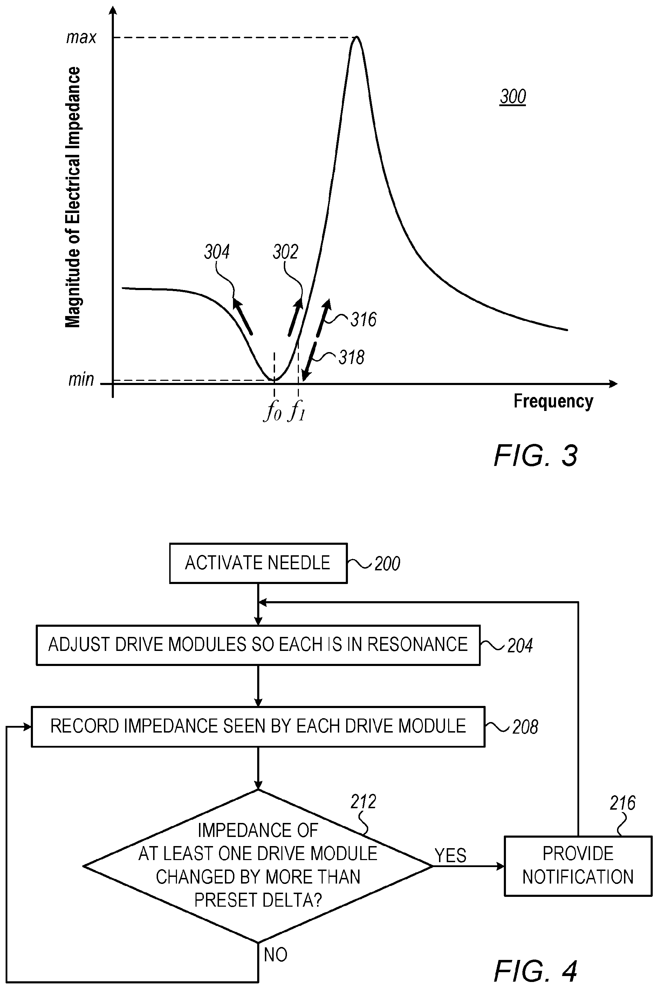

Moreover, an extension of the method is provided, in which the driving frequency is slightly detuned from being at a minimum of an electrical impedance magnitude curve to a slope of the curve, as shown by driving frequency detuned from $f_0$ to $f_1$ in Fig. 3 (i.e., tuned slightly off-resonance), in order to generate a more distinctive (i.e., in inverse direction) change in impedance, examples being a drop in electrical impedance with increased mechanical load, and a rise in electrical impedance with reduced load. Using such a detuned frequency, a processor can, for example, indicate more reliably if a tip of the probe is engaging or disengaging with an eye, or if a viscosity of media in which the tip is vibrating increases or decreases.

SYSTEM DESCRIPTION

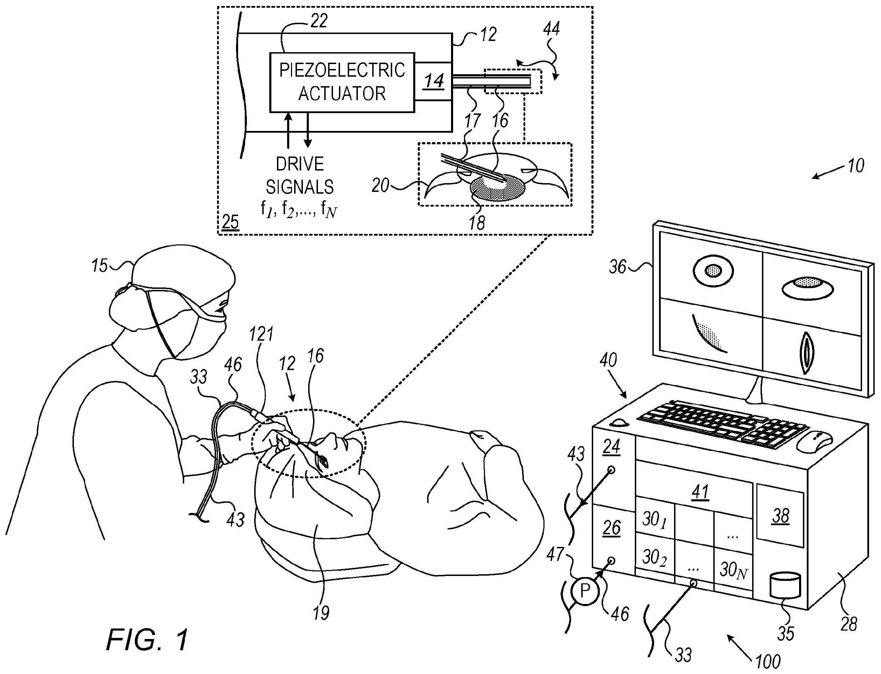

Fig. 1 is a pictorial view of a phacoemulsification system 10 constructed to operate in accordance with an example of the present disclosure. Fig. 1 includes an inset 25, and, as shown in the figure and the inset system 10, includes a phacoemulsification probe/handpiece 12 comprising a needle 16. Needle 16 is configured to be inserted into a lens capsule 18 of an eye 20 of a patient 19. Needle 16 is mounted on a horn 14 of probe 12 and is shown in inset 25 as a straight needle. However, any suitable needle may be used with phacoemulsification probe 12, for example, a curved or bent tip needle commercially available from Johnson & Johnson Surgical Vision, Irvine, CA, USA. A physician 15 holds handpiece 12 by a handle 121 so as to perform a phacoemulsification procedure on the eye of patient 19. The physician may activate the handpiece using a foot pedal (not illustrated in Fig. 1).

Handpiece 12 comprises a piezoelectric actuator 22, which is configured to vibrate horn 14 and needle 16 in one or more resonant vibration modes of the combined horn and needle element. During the phacoemulsification procedure, the vibration of needle 16 is used to break a cataract into small pieces.

During the phacoemulsification procedure, an irrigation sub-system 24, which may be located in a console 28, pumps irrigation fluid from an irrigation reservoir to an irrigation sleeve 17 surrounding at least a portion of needle 16, so as to irrigate the eye. The fluid is pumped via a tubing line 43 running from the console 28 to the probe 12. Irrigation sub-system 24 is described in more detail below.

An aspiration sub-system 26, also typically located in console 28, aspirates eye fluid and waste matter (e.g., emulsified parts of the cataract) from the patient's eye via needle 16 to a collection receptacle (not shown). Aspiration sub-system 26 comprises a pump which produces a vacuum that is connected from the sub-system to probe 12 by a vacuum tubing line 46. A gauge or sensor 47 in line 46 measures the aspiration vacuum pressure. Gauge 47 may be in any convenient location in line 46, including, but not limited to, a location in or in proximity to handpiece 12 or a location in or in proximity to the console.

Irrigation sub-system 24 and aspiration sub-system 26 are both controlled by a processor 38. The processor controls the flow volume rate at which the irrigation sub-system pumps fluid. The processor also controls the vacuum pressure produced by the aspiration sub-system, using a pressure reading from gauge 47.

Some or all of the functions of processor 38 may be combined in a single physical component or, alternatively, implemented using multiple physical components. The physical components may comprise hard-wired or programmable devices, or a combination of the two. In some examples, at least some of the functions of processor 38 may be carried out by suitable software stored in a memory 35. The software may be downloaded to a device in electronic form, over a network, for example. Alternatively, or additionally, the software may be stored in tangible, non-transitory computer-readable storage media, such as optical, magnetic, or electronic memory.

Processor 38 may receive user-based commands via a user interface 40, which may include setting and/or adjusting a vibration mode and/or a frequency of piezoelectric actuator 22, setting and/or adjusting a stroke amplitude of needle 16, and setting and/or adjusting an irrigation rate and an aspiration rate of irrigation sub-system 24 and aspiration sub-system 26. Additionally, or alternatively, processor 38 may receive user-based commands from controls located in handpiece 12, to, for example, select a trajectory 44, or another trajectory, for needle 16. The implementation of a trajectory such as trajectory 44 is described further below.

Processor 38 may present results of the phacoemulsification procedure on a display 36. In an example, user interface 40 and display 36 may be one and the same, such as a touch screen graphical user interface.

The system illustrated in Fig. 1 may include further elements, which are omitted for clarity of presentation. For example, physician 15 typically performs the procedure using a stereo-microscope or magnifying glasses, neither of which are shown. Physician 15 may use other surgical tools, in addition to probe 12, which are also not shown to maintain clarity and simplicity.

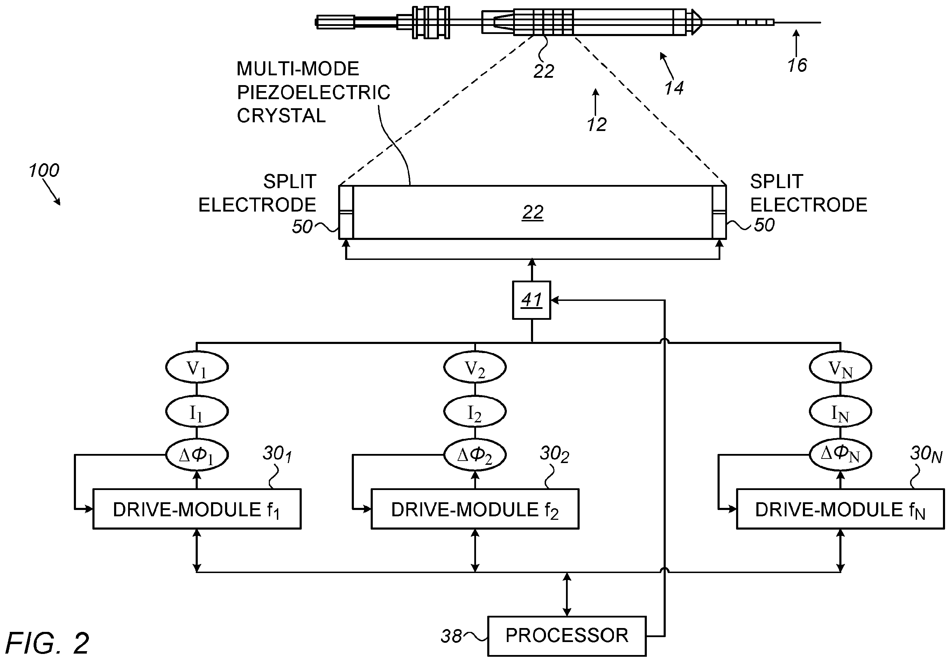

Console 28 further comprises a multi-channel piezoelectric drive system 100 comprising drive-modules $30_1$, $30_2$, ... $30_N$, each coupled, using wiring in a cable 33, to a stack of piezoelectric crystals of actuator 22. Drive-modules $30_1$, $30_2$, ... $30_N$, generically termed drive-modules 30, are controlled by processor 38 and convey phase-controlled driving signals via cable 33 to piezoelectric actuator 22. In response, piezoelectric actuator 22 vibrates needle 16, which performs a vibrational/ultrasound trajectory 44, the trajectory typically comprising one or a combination of longitudinal, transverse, and/or torsional ultrasonic vibrations synchronized one with the other. System 100 is described further below, with reference to Fig. 2.

## PIEZOELECTRIC RESONANT SYSTEM FOR PHACOEMULSIFICATION PROBE

Fig. 2 is a block diagram schematically illustrating multi-channel piezoelectric drive system 100, in accordance with an example of the present disclosure.

As stated above, drive system 100 comprises drive-modules $30_1$, $30_2$, ... $30_N$, each of which may be coupled to one or more split electrodes 50 of piezoelectric actuator 22 (which may comprise a multi-stack crystal) of phacoemulsification probe 12, using electrical links running in cable 33. Each drive module 30 is configured to generate respective different frequencies $f_1$, $f_2$, ... $f_N$, and processor 38 is able to measure the impedance seen by the module 30 using signals from respective potential meters $V_1$, $V_2$, ... $V_N$, and current meters $I_1$, $I_2$, ... $I_N$. In addition, processor 38 is able to measure the phase differences $\Delta\phi_j$, j=1,2..., N of each of the drive signals generated by the modules.

Processor 38 adjusts the different frequencies $f_1$, $f_2$, ... $f_N$ of the drive signals to minimize measured phase differences using any suitable method, for example, an optimization algorithm, which is not limited to a gradient descent algorithm. An apparatus that can adjust a frequency of a drive signal so as to minimize the measured phase difference, thereby maintaining a piezoelectric actuator vibrating at a resonant frequency, is described in U.S. Patent Application 16/727,100, filed December 26, 2019, titled "Phacoemulsification Apparatus," which is assigned to the assignee of the present patent application. U.S. Patent Application 16/727,100 also describes generation of different modes of vibration.

In an alternative example (not shown), the actuators are controlled by processor 38, by, for example, minimizing impedances $z_1$, $z_2$, ... $z_N$ experienced by the drive system in the multi-resonance mode.

The example illustration shown in Fig. 2 is chosen purely for the sake of conceptual clarity. Fig. 2 shows only parts relevant to examples of the present disclosure. While Fig. 2 shows a multi-mode/multi-frequency scheme, the disclosure is just as applicable to phacoemulsification probes driven by a single-mode/single-frequency driving signal (e.g., to phacoemulsification probes that vibrates in one mode).

## DETECTING PHACOEMULSIFICATION TIP ENGAGEMENT WITH LENS

As noted above, the electrical response of an ultrasound transducer seen by a driving circuitry is dominated by an impedance minimum near the mechanical resonance frequency $f_0$, as seen in Fig. 3 below.

Fig. 3 is a schematic graph 300 showing the electrical impedance magnitude $|z(f)|$ of an ultrasonic transducer as a function of driving frequency, f, in accordance with an example of the present disclosure. The shown electrical response curve of $|z(f)|$ is for a given mechanical load. As seen, the electrical impedance magnitude has a resonant line shape where impedance is minimal at $f_0$ at the given mechanical load. If the mechanical load increases, the curve shifts to the left (to lower resonant frequency according to Eq. 1 below). If the mechanical load decreases, the curve shifts to the right (to higher resonant frequency according to Eq. 1 below). In any case, the measured impedance rises from that minimum, i.e.,

whether the mechanical load on the vibrating element is increased (302) or decreased (304). Thus, any change in load on a well-tuned driving circuitry can be detected. Moreover, detuning the driving frequency, for example to a frequency $f_1$, with $f_1$ being located on a slope of the curve, so it can be utilized to generate a distinctive change (e.g., rise (316) in impedance with increased mechanical load, and a drop (318) in impedance with a reduced load). Using a detuned frequency, such as $f_1$, a processor can indicate, for example, if a tip of the probe is engaging or disengaging with an eye, or if a viscosity of media in which the tip is vibrating increases or decreases.

As can be seen, it may be difficult for physician 15 to know when the needle inserted into the patient's eye has made contact with the eye lens, without, for example, the system measuring the impedance during the phacoemulsification procedure.

Fig. 4 is a flow chart describing steps providing physician 15 with an indication that needle 16 has contacted the eye lens of patient 19, in accordance with an example of the present disclosure. The method of the flow chart is based on the equation of mechanical resonance of a mass (e.g., a mechanical load) on a spring (e.g., harmonic oscillator):

$$f_0 = \frac{1}{2\pi} \sqrt{\frac{K}{m}} \tag{1}$$

where $f_0$ is the resonant frequency of the spring, K is the spring constant of the spring, and m is the mass on the spring.

In examples of the present disclosure, needle 16 may be considered to correspond to a spring, and the material contacting the end of the needle may be considered to correspond to an effective mass on the spring. It will be understood that as the needle contacts different materials in the eye, the mechanical resonant frequency of the needle changes.

In an initial step 200 of the flow chart, physician 15 activates handpiece 12 and decides on a trajectory that the needle of the handpiece is to follow, or the trajectory is preprogrammed. By way of example, the physician is assumed to decide on using a longitudinal trajectory, and the physician inputs an appropriate notification to processor 38.

In step 204, the processor 38 decides which drive modules are to be activated and adjusted. The processor 38 adjusts the frequency and phase of each of the activated modules 30 so that each is in electrical resonance, i.e., the impedance seen by each module, measured using its potential meter V and current meter I (Fig. 2), is a minimum. It will be understood that the adjustment performed in step 204 is a dynamic process, since, for example, the adjustments for one drive module may affect adjustments needed for another drive module. Furthermore, handpiece 12 and its elements, including the piezoelectric crystal, may be subject to other physical effects, including temperature changes, which may alter the electrical resonant frequencies of the drive modules.

In a recording step 208, processor 38 registers the values of impedance seen by each module.

In a decision step 212, processor 38 checks if the impedance seen by any one of the drive modules has changed by more than a preset amount, as derived, for example, from calibration and stored in a look up table. It will be appreciated that if the mechanical resonant frequency of needle 16 changes, this affects the electrical impedances seen by the drive modules. In a disclosed example, the preset impedance change in step 212 is selected to be an increase in electrical impedance that reflects the mechanical resonant frequency decrease that occurs when the needle begins to engage with the eye lens due to the increase of effective mass on the needle. In an example, if the trajectory comprises a longitudinal mode of vibration, the preset impedance change is approximately 1 k$\Omega$, and if the trajectory comprises a torsional mode of vibration, the preset impedance change is approximately 5 k$\Omega$.

If step 212 returns negative ("No"), i.e., none of the drive modules see an impedance change greater than the preset amount, control returns to recording step 208 so that the step reiterates.

If step 212 returns positive ("Yes"), i.e., at least one of the drive modules sees an impedance change greater that the preset amount, control continues to a notification step 216, and from the notification step control returns to adjust step 204.

In notification step 216 processor 38 provides an appropriate notification based on the change of decision step 212. For example, in the case of the disclosed example described above, the notification may comprise text on display 36 stating that the needle has begun to engage with the eye lens or an audible sound may be made indicating the same. In addition to notifying of the change to the handpiece user, the processor may change a driving signal property in response to the change, such as reducing vibration power to lower a level of vibration of the needle when disengagement from the lens is detected.

By appropriate choice of values of the preset impedance change in step 212, physician 15 may be notified of other transitions of needle 16 within an eye of patient 19. Such transitions include disengagement of the needle from the lens, wherein the preset impedance change is a decrease in electrical impedance. Other transitions of needle 16, for example through regions of the eye with different viscosities, may be detected using other values of the preset impedance change, and the other values may be determined, *mutatis mutandis*, by one with ordinary skill in the art without undue experimentation.

During a phacoemulsification procedure, there is a large number of parameters that the physician may select when performing the procedure in order to control the ultrasonic trajectory of needle 16. In addition to controlling the trajectory, i.e., the path followed by a distal end of the needle, the processor, or the physician, also controls the power delivered (which typically affects the amplitude of the ultrasonic vibration) as well as the duration of the trajectory.

The example flow chart shown in Fig. 4 is chosen purely for the sake of conceptual clarity. For example, additional steps such as irrigating the eye and aspiring cataract lens particles are omitted for simplicity and clarity of presentation.

Although the examples described herein mainly address phacoemulsification, the methods and systems described herein can also be used in other surgical applications that may require a multi-channel piezoelectric resonant system to drive a moving member, such as ultrasonic blades, and other types of actuators.

It will thus be appreciated that the examples described above are cited by way of example, and that the present disclosure is not limited to what has been particularly shown and described hereinabove. Rather, the scope of the present disclosure includes both combinations and sub-combinations of the various features described hereinabove, as well as variations and modifications thereof which would occur to persons skilled in the art upon reading the foregoing description and which are not disclosed in the prior art.

## Claims

1. A system (10), comprising:

   a phacoemulsification handpiece (12) comprising a needle (16) and one or more piezoelectric crystals (22), the needle being configured to be inserted into an eye (20) of a patient and be vibrated by driving the one or more piezoelectric crystals (22) in the handpiece with one or more electrical signals having different respective frequencies using one or more respective drive modules; and
   a processor (38), which is configured to:

   tune the frequencies of the electrical signals to respective one or more target frequencies, and register respective electrical impedances seen by the drive modules, wherein the processor is configured to tune the frequencies of the electrical signals to respective one or more target frequencies by detuning one or more of the frequencies off-resonance, in order to have a decrease in electrical impedance magnitude with an increased mechanical load on the needle; and
   in response to an electrical impedance seen by at least one of the drive modules undergoing a change exceeding a preset impedance change, provide an indication that material in the eye surrounding the needle has changed.

2. The system according to claim 1, wherein the processor is further configured to, in response to an electrical impedance seen by at least one of the drive modules undergoing a change, change a level of vibration of the needle.

3. The system according to claim 1, wherein the processor is configured to tune the one or more frequencies by setting the target frequencies to be respective resonance frequencies of the one or more piezoelectric crystals as measured by the drive modules.

4. The system according to claim 3, wherein tuning the frequencies comprises setting the target frequencies to be respective resonance frequencies of the one or more piezoelectric crystals as measured by the drive modules, so as to differentiate between the needle engaging with the material, and the needle disengaging from the material.

5. The system according to claim 4, wherein the material is lens material.

6. The system according to claim 1, wherein the change in the impedance is in response to one of the needle engaging with the material of the eye, and the needle disengaging from the material of the eye.

7. The system according to claim 1, wherein the processor is configured to provide an indication by providing one of an audio and visual indication.

8. The system according to claim 1, wherein the processor is configured to provide an indication by providing vibratory indication.

9. The system according to claim 1, wherein the material is lens material.

**10.** A system, comprising:

a phacoemulsification handpiece comprising a needle (16) and one or more piezoelectric crystals (22), the needle being configured to be inserted into an eye of a patient and be vibrated by driving the one or more piezoelectric crystals in the handpiece with one or more electrical signals having different respective frequencies using one or more respective drive modules; and
a processor, which is configured to:

tune the frequencies of the electrical signals to respective one or more target frequencies, and register respective electrical impedances seen by the drive modules, wherein the processor is configured to tune the frequencies of the electrical signals to respective one or more target frequencies by detuning one or more of the frequencies off-resonance, in order to have a decrease in electrical impedance magnitude with an increased mechanical load on the needle; and
in response to an electrical impedance seen by at least one of the drive modules undergoing a change exceeding a preset impedance change, change a level of vibration of the needle.


**Patentansprüche**

**1.** System (10), umfassend:

ein Phakoemulsifikationshandstück (12), umfassend eine Nadel (16) und einen oder mehrere piezoelektrische Kristalle (22), wobei die Nadel konfiguriert ist, um in ein Auge (20) eines Patienten eingeführt zu werden und durch Antreiben des einen oder der mehreren piezoelektrischen Kristalle (22) in dem Handstück mit einem oder mehreren elektrischen Signalen mit unterschiedlichen jeweiligen Frequenzen unter Verwendung eines oder mehrerer jeweiliger Antriebsmodule in Schwingung versetzt zu werden; und
einen Prozessor (38), der konfiguriert ist zum:

Abstimmen der Frequenzen der elektrischen Signale auf jeweilige eine oder mehrere Zielfrequenzen, und Registrieren jeweiliger elektrische Impedanzen, die von den Antriebsmodulen gesehen werden, wobei der Prozessor konfiguriert ist, um die Frequenzen der elektrischen Signale auf jeweilige eine oder mehrere Zielfrequenzen abzustimmen, indem eine oder mehrere der Frequenzen außerhalb der Resonanz verstimmt werden, um eine Abnahme der elektrischen Impedanzgröße bei einer erhöhten mechanischen Belastung der Nadel zu erreichen; und
als Reaktion auf eine elektrische Impedanz, die von mindestens einem der Antriebsmodule wahrgenommen wird, die eine Änderung erfährt, die eine voreingestellte Impedanzänderung überschreitet, Bereitstellen einer Anzeige, dass sich Material im Auge um die Nadel herum verändert hat.

**2.** System nach Anspruch 1, wobei der Prozessor ferner konfiguriert ist, um als Reaktion auf eine elektrische Impedanz, die von mindestens einem der Antriebsmodule gesehen wird, die eine Änderung erfährt, eine Vibrationsstärke der Nadel zu ändern.

**3.** System nach Anspruch 1, wobei der Prozessor konfiguriert ist, um die eine oder mehreren Frequenzen durch Einstellen der Zielfrequenzen auf jeweilige Resonanzfrequenzen der einen oder mehreren piezoelektrischen Kristalle, wie durch die Antriebsmodule gemessen, abzustimmen.

**4.** System nach Anspruch 3, wobei das Abstimmen der Frequenzen das Einstellen der Zielfrequenzen auf jeweilige Resonanzfrequenzen der einen oder mehreren piezoelektrischen Kristalle, wie von den Antriebsmodulen gemessen, umfasst, um zwischen dem Eingriff der Nadel in das Material und dem Lösen der Nadel von dem Material zu unterscheiden.

**5.** System nach Anspruch 4, wobei das Material Linsenmaterial ist.

**6.** System nach Anspruch 1, wobei die Änderung der Impedanz als Reaktion auf eines von dem Eingriff der Nadel in das Material des Auges und dem Lösen der Nadel von dem Material des Auges erfolgt.

**7.** System nach Anspruch 1, wobei der Prozessor konfiguriert ist, um eine Anzeige durch Bereitstellen einer von einer akustischen und einer visuellen Anzeige bereitzustellen.

8. System nach Anspruch 1, wobei der Prozessor konfiguriert ist, um eine Anzeige durch Bereitstellen einer Vibrationsanzeige bereitzustellen.

9. System nach Anspruch 1, wobei das Material Linsenmaterial ist.

10. System, umfassend:

ein Phakoemulsifikationshandstück, das eine Nadel (16) und einen oder mehrere piezoelektrische Kristalle (22) umfasst, wobei die Nadel konfiguriert ist, um in ein Auge eines Patienten eingeführt zu werden und durch Antreiben des einen oder der mehreren piezoelektrischen Kristalle in dem Handstück mit einem oder mehreren elektrischen Signalen, die unterschiedliche jeweilige Frequenzen aufweisen, unter Verwendung eines oder mehrerer jeweiliger Antriebsmodule in Schwingung versetzt zu werden; und
einen Prozessor, der konfiguriert ist zum:

Abstimmen der Frequenzen der elektrischen Signale auf jeweilige eine oder mehrere Zielfrequenzen, und Registrieren jeweiliger elektrischer Impedanzen, die von den Antriebsmodulen gesehen werden, wobei der Prozessor konfiguriert ist, um die Frequenzen der elektrischen Signale auf jeweilige eine oder mehrere Zielfrequenzen abzustimmen, indem eine oder mehrere der Frequenzen außerhalb der Resonanz verstimmt werden, um eine Abnahme der elektrischen Impedanzgröße bei einer erhöhten mechanischen Belastung der Nadel zu erreichen; und
Ändern eines Schwingungsgrades der Nadel als Reaktion auf eine elektrische Impedanz, die von mindestens einem der Antriebsmodule wahrgenommen wird und eine Änderung erfährt, die eine voreingestellte Impedanzänderung überschreitet.

## Revendications

1. Système (10), comprenant :

une pièce à main de phacoémulsification (12) comprenant une aiguille (16) et un ou plusieurs cristaux piézoélectriques (22), l'aiguille étant conçue pour être insérée dans un œil (20) d'un patient et être mise en vibration en pilotant le ou les cristaux piézoélectriques (22) dans la pièce à main avec un ou plusieurs signaux électriques ayant différentes fréquences respectives à l'aide d'un ou plusieurs modules de pilotage respectifs ; et
un processeur (38) qui est configuré pour :

accorder les fréquences des signaux électriques sur une ou plusieurs fréquences cibles respectives, et enregistrer les impédances électriques respectives observées par les modules de pilotage, dans lequel le processeur est configuré pour accorder les fréquences des signaux électriques à la ou aux fréquences cibles respectives en désaccordant une ou plusieurs des fréquences hors résonance, afin d'avoir une diminution de grandeur d'impédance électrique avec une charge mécanique augmentée sur l'aiguille ; et
en réponse à une impédance électrique observée par au moins l'un des modules de pilotage subissant un changement dépassant un changement d'impédance préréglé, fournir une indication que le matériau dans l'œil entourant l'aiguille a changé.

2. Système selon la revendication 1, dans lequel le processeur est configuré en outre pour, en réponse à une impédance électrique observée par au moins l'un des modules de pilotage subissant un changement, changer un niveau de vibration de l'aiguille.

3. Système selon la revendication 1, dans lequel le processeur est configuré pour accorder la ou les fréquences en réglant les fréquences cibles pour qu'elles soient les fréquences de résonance respectives du ou des cristaux piézoélectriques telles que mesurées par les modules de pilotage.

4. Système selon la revendication 3, dans lequel le fait d'accorder les fréquences comprend le réglage des fréquences cibles pour qu'elles soient les fréquences de résonance respectives du ou des cristaux piézoélectriques telles que mesurées par les modules de pilotage, de façon à faire la différence entre l'aiguille pénétrant dans le matériau, et l'aiguille ressortant du matériau.

5. Système selon la revendication 4, dans lequel le matériau est un matériau cristallinien.

6. Système selon la revendication 1, dans lequel le changement dans l'impédance est en réponse à l'un parmi l'aiguille pénétrant dans le matériau de l'œil, et l'aiguille ressortant du matériau de l'œil.

7. Système selon la revendication 1, dans lequel le processeur est configuré pour fournir une indication en fournissant l'une parmi une indication audio et visuelle.

8. Système selon la revendication 1, dans lequel le processeur est configuré pour fournir une indication en fournissant une indication vibratoire.

9. Système selon la revendication 1, dans lequel le matériau est un matériau cristallinien.

10. Système, comprenant :

une pièce à main de phacoémulsification comprenant une aiguille (16) et un ou plusieurs cristaux piézoélectriques (22), l'aiguille étant conçue pour être insérée dans un œil d'un patient et être mise en vibration en pilotant le ou les cristaux piézoélectriques dans la pièce à main avec un ou plusieurs signaux électriques ayant différentes fréquences respectives à l'aide d'un ou plusieurs modules de pilotage respectifs ; et
un processeur qui est configuré pour :

accorder les fréquences des signaux électriques sur une ou plusieurs fréquences cibles respectives, et enregistrer les impédances électriques respectives observées par les modules de pilotage, dans lequel le processeur est configuré pour accorder les fréquences des signaux électriques à la ou aux fréquences cibles respectives en désaccordant une ou plusieurs des fréquences hors résonance, afin d'avoir une diminution de grandeur d'impédance électrique avec une charge mécanique augmentée sur l'aiguille ; et
en réponse à une impédance électrique observée par au moins l'un des modules de pilotage subissant un changement dépassant un changement d'impédance préréglé, changer un niveau de vibration de l'aiguille.

*FIG. 1*

FIG. 2

FIG. 3

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021119616 A
- WO 9211814 A1
- US 72710019
- US 727100